# EUROPEAN PATENT APPLICATION

(11) **EP 2 604 258 A1**
(43) Date of publication of application: **19.06.2013**
(21) Application number: 12161644.5
(22) Date of filing: 28.03.2012
(51) Int. Cl.: A61K 31/00, A61K 31/351, A61K 31/402, A61K 31/4178, A61P 21/00

(54) **Pharmaceutical composition comprising Losartan for treating or preventing statin based drug-induced muscle toxicity**

(30) Priority: 16.12.2011 KR 20110136687
(71) Applicant: HanAll Biopharma Co., Ltd., Daejeon 306-120 (KR)
(72) Inventor: Kim, Sung Wuk, 306-120 Daejeon (KR); Choe, Seong Choon, 306-120 Daejeon (KR); Min, Chang Hee, 306-120 Daejeon (KR); Lee, Soon IM, 306-120 Daejeon (KR); Koo, Ja Seong, 306-120 Daejeon (KR); Sun, Sang Ouk, 306-120 Daejeon (KR); Lee, Na Young, 306-120 Daejeon (KR)
(74) Representative: Vossius & Partner

(57) **Abstract**

A pharmaceutical composition comprising losartan or a pharmaceutically acceptable salt thereof, which is capable of treating or preventing side effects such as muscle toxicity caused by administering a statin-based lipid-lowering drug, and a pharmaceutical combination composition comprising a statin drug and a losartan drug as active ingredients are provided. The pharmaceutical composition comprising losartan or a pharmaceutically acceptable salt thereof provides an effect on treating or preventing muscle-related side effects caused by administration of a statin drug for treating hyperlipidemia, as well as an intrinsic pharmacological effect for treating or preventing hypertension. When administered along with a statin drug in sequence or co-administered at the same time with the statin drug, the pharmaceutical composition can exhibit an effect of effectively or significantly decreasing or preventing side effects such as muscle toxicity.

## Description

### CROSS-REFERENCE TO RELATED APPLICATION

This application claims priority to and the benefit of Korean Patent Application No. 2011-0136687, filed December 16, 2011, the disclosure of which is incorporated herein by reference in its entirety.

### BACKGROUND

### 1. Field of the Invention

The present invention relates to a pharmaceutical composition comprising losartan or a pharmaceutically acceptable salt thereof, for treating or preventing muscle toxicity, i.e., a side effect that may be caused by the administration of a statin-based lipid-lowering agent, and a pharmaceutical combination composition comprising a statin drug and a losartan drug as an active ingredient.

### 2. Discussion of Related Art

Hyperlipidemia refers to the state of having abnormally increased lipids, such as cholesterol or triglycerides in plasma due to a genetic factor, excessive consumption of animal fats and carbohydrates, obesity, diabetes, renal diseases, hypothyroidism, and the like. Especially, hyperlipidemia induces arterial thrombosis, thereby causing arteriosclerosis, in which lipids are thickly stacked up along the walls of the arteries, so that blood flow is decreased, causing ischemic heart disease, angina, and myocardial infarctions. Accordingly, other cardiovascular diseases such as arteriosclerosis can be prevented by treating hyperlipidemia.

A representative drug for treating hyperlipidemia as mentioned above is the statin-based lipid-lowering drug inhibiting cholesterol biosynthesis by inhibiting HMG-CoA reductase [Lancet 1995; 346:750-753, Am J Cardiol 1998; 82: 57T-59T, Am J Cardiol 1995; 76: 107C-112C, Hypertension Res 2003; 26: 699-704]. The statin-based lipid-lowering drug works by inhibiting the activity of HMG-CoA reductase involved in the process of converting HMG-CoA into mevalonic acid, which is a step of determining the biosynthesis rate of cholesterol (rate determining step) in the hepatocytes.

The statin-based lipid-lowering drugs include simvastatin, lovastatin, atorvastatin, pravastatin, fluvastatin, rosuvastatin, pitavastatin and pharmaceutically acceptable salts thereof, and atorvastatin, simvastatin, and rosuvastatin are the most widely used. Especially, the most representative drug is atorvastatin represented by the following Formula 1, and salts thereof.

Formula 1

Structure of Atorvastatin

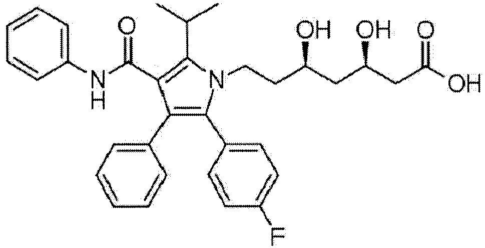

However, the statin-based lipid-lowering drugs have a risk of generating a great side effect (toxicity) in the skeletal muscles. The representative side effects in the skeletal muscles may include myositis, myopathy, rhabdomyolysis, myalgia, and the like. The exact causes of the side effects remain unknown. However, it is known that since atrogin-1 gene is induced in the case of statin-induced muscular side effects, atrogin-1 gene is closely involved as its mediator when a muscle is injured due to the statin. [The muscle-specific ubiquitin ligase atrogin-1/MAFbx mediates statin-induced muscle toxicity. (2007)]

In addition, it is reported that the side effects are increased when the concentration of the statin-based drug in blood is increased in the most cases. For example, the frequency in percentage to cause muscular side effects according to the dosage of the drug simvastatin is 0.03% when taking 20 mg per day and 0.08% when taking 40 mg per day, but 0.61% when taking 80 mg per day, so it can be seen that there is an increaseof at least about 20 times in frequency for a dosage of 80 mg compared to a dosage of 20 mg [Zocor Tablet Label, FDA Oct 16, 2011].

Muscular side effects from increasing the concentration of a statin-based drug in the blood are due to the interaction between the drugs when administered in combination with other drugs. When metabolizing enzymes (representatively, cytochrome P450-based enzyme) or transporters (representatively, Pgp transporter) used for absorption or excretion of one drug is not normally used with another co-absorbed drug, the concentration in the blood is increased or decreased, which is called a drug-drug interaction. When the concentration of the statin-based drug in the blood is abnormally increased due to the drug-drug interaction, muscular side effects may occur with an increased concentration of creatine kinase (hereinafter, CK). Representative drugs known to generate an interaction with a statin-based drug include fibric acid derivatives, cyclosporin, erythromycin, clarithromycin, niacin, and the like [Zocor Tablet Label, FDA Oct 16, 2011. Lipitor Tablet Label, FDA Jun 17, 2009. Crestor Tablet, FDA Nov 19, 2010.].

Losartan, represented by the following Formula 2, is 2-butyl-4 chloro-1-[2-(1H-tetrazol-5-yl)biphenyl-4-ylphenyl]-1H-imidazol-5-methanol and an anti-hypertension drug that antagonizes the bonding of angiotensin-II (AII) to a receptor in the walls of the arteries (AII receptor) [Korean Patent Publication No. 1990-0005045]. The angiotensin-II is a factor that induces raised blood pressure, left ventricular hypertrophy, vascular hypertrophy, atherosclerosis, renal insufficiency, stroke, and the like.

Formula 2

Structure of Losartan

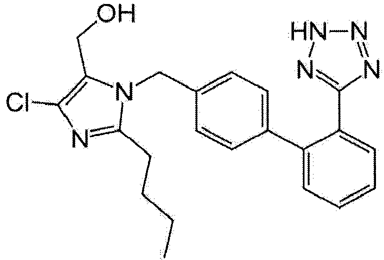

Losartan is a drug that has a function of lowering blood pressure, and also plays wide roles in prevention and treatment of cardiac insufficiency, prevention and treatment of arrhythmia and cardiac insufficiency after the onset of myocardial infarction, prevention and treatment of diabetic complications, prevention and treatment of renal insufficiency, prevention and treatment of strokes, induction of anti-platelet effects, prevention of sclerosis in the arteries, inhibition of adverse action of aldosterone, prevention of serial deterioration of disorders in the circulatory system, and the like [J. Hypertens., vol. 13(8) (1995), p.891-899, Kidney Int., vol.57(2)(2000), p.601-606, Circulation, vol. 101(14) (2000), p.1653-1659, J. Hypertension., vol. 17(7) (1999), p.907-916].

It is already a well-known fact that an angiotensin-II antagonist, such as losartan, is an AMPK (AMP-activated protein kinase) activator. However, research has suggested that the AMPK activator increases expression of the atrogin-1 gene, which is the main marker of the statin-based muscular side effects [AMP-activated protein kinase agonists increase mRNA content of the muscle-specific ubiquitin ligases MAFbx and MuRF1 in C2C12 cells (2007)], and thus statin's muscular side effects can be expected to further increase due to the increase in atrogin-1 gene when statin and losartan are used together.

In addition, when the losartan drug is absorbed after oral administration, it first flows into the liver. Some of it is ingested as losartan molecules into the blood, reaching the maximum concentration in the blood within one hour and some of it is metabolized by a 2C9 enzyme and a 3A4 enzyme among cytochrome P450 enzymes present in the liver, and converted into an active metabolite having a higher pharmacological action, exhibiting the maximum concentration in the blood within 3 to 4 hours. In such absorption and excretion processes, the losartan drug uses a transporter like the drug statin. Accordingly, it is known that a concentration of a statin-based drug in the blood is increased due to a competitive interaction with the cytochrome metabolic enzymes and the transporter when the losartan drug and the statin-based drug are administered at the same time [J. Bioequivalence & Bioavailability (1) p. 18-27, 2009]. The increased statin concentration in the blood increases the possibility of causing muscle toxicity as a side effect of the statin.

The known effects involved in the new losartan drug that exhibits a decreased effect in side effects of the statin proposed in the present invention are as follows.

US Registration Patent No. 5153197 discloses an effect of losartan on treating hypertension.

Korean Registration Patent No. 0692235 discloses an effect of a drug candesartan, which is the same ARB-based drug, on vascular headache symptoms.

Korean Publication Patent No. 1999-014905 discloses an effect of ARB-based drug on multiple organ dysfunction.

However, all of the above-described techniques differ from the technique of the present invention, providing a pharmaceutical composition of losartan, or a pharmaceutically acceptable salt thereof, which can treat and prevent muscle toxicity generated due to a statin drug.

### SUMMARY OF THE INVENTION

The present invention is directed to providing a new pharmaceutical composition that can treat or prevent muscular side effects, such as myositis, myopathy, rhabdomyolysis, and myalgia, which are caused by administering a statin-based lipid-lowering drug.

One aspect of the present invention provides a new use of losartan or a pharmaceutically acceptable salt thereof for treating or preventing muscular side effects, such as myositis, myopathy, rhabdomyolysis, and myalgia, which are caused by administering a statin-based lipid-lowering drug.

Another aspect of the present invention provides a pharmaceutical combination composition of losartan and a statin-based drug for decreasing lipid contents while more effectively treating and preventing muscular side effects of the statin-based lipid-lowering agent when the composition comprises losartan and the statin-based drug as active ingredients.

Hereinafter, the effects of losartan or a pharmaceutically acceptable salt thereof on treating and preventing muscle toxicity as a side effect and a pharmaceutical composition containing the same will be described according to a specific embodiment of the present invention.

Generally, a patient who is taking the statin-based lipid-lowering drug to with the object of treating hyperlipidemia has a high probability for combination treatment with other drugs due to other chronic diseases. For this reason, the concentration of the statin-based drug in the blood is increased, and in particular, an expression of the astrogin-1 gene is induced in the muscles to cause muscular side effects.

The losartan drug was originally used to prevent or treat hypertension. Losartan or a pharmaceutically acceptable salt thereof may be expected to increase the concentration of the statin-based drug in the blood due to a competitive interaction of the cytochrome enzyme or transporter with the statin-based drug, and also may increase the muscular side effects by inducing the expression of the atrogin-1 gene, which is a main marker of muscle damage.

As seen from the following examples, however, it can be surprisingly confirmed that muscular side effects may be significantly decreased and be prevented unexpectedly by administering the losartan drug, which is known as a drug for treating hypertension during the treatment with the statin-based lipid-lowering drug.

Therefore, the pharmaceutical composition comprising losartan or a pharmaceutically acceptable salt thereof as an active ingredient according to the present invention can provide a first medicinal use for treating or preventing hypertension and a second medicinal use for treating or preventing muscular side effects due to a statin-based lipid-lowering drug at the same time.

In order to exhibit the second medicinal use of losartan or a pharmaceutically acceptable salt thereof for treating or preventing muscular side effects due to the statin-based lipid-lowering drug, it is based on the assumption that losartan or the pharmaceutically acceptable salt thereof should be administered with a statin-based lipid-lowering drug at the same time or in sequence.

The expression "muscular side effects due to a statin-based lipid-lowering drug" throughout the specification refers to at least one of muscle-related side effects, such as myositis, myopathy, rhabdomyolysis, myalgia, and the like which are caused by administering a statin-based lipid-lowering drug.

For the pharmaceutical composition of the present invention, the pharmaceutically acceptable salt of losartan may be potassium salts, magnesium salts, calcium salts, sodium salts, and the like, but the present invention is not limited thereto. Here, losartan potassium salts is preferred.

Meanwhile, for the pharmaceutical composition of the present invention, the statin-based lipid-lowering drug may include at least one selected from the group consisting of simvastatin, lovastatin, atorvastatin, pravastatin, pitavastatin, rousuvastatin, fluvastatin and pharmaceutically acceptable salts thereof. In addition, the term "pharmaceutical composition" refers to a composition comprising the statin-based lipid-lowering drug only or a composition obtained by properly mixing the statin-based lipid-lowering drug with other pharmaceutically acceptable carriers, such as a binder, filler, disintergrant, surfactant, lubricant, dispersant, buffer solution, preservative, flavor, fragrance, coating agent, diluent, and the like, for the purpose of oral or parenteral administration, unless otherwise indicated.

The pharmaceutical composition comprising losartan and the pharmaceutically acceptable salt thereof of the present invention also includes a combination composition formulated to be taken with the statin-based lipid-lowering drug at the same time in order to prevent side effects, as well as a single dosage type administered to treat or prevent muscular side effects due to the statin-based lipid-lowering drug.

Accordingly, the present invention provides a pharmaceutical composition for decreasing lipids and for treating or preventing muscular side effects due to the statin-based lipid-lowering drug, comprising losartan or a pharmaceutically acceptable salt thereof, and the statin-based lipid-lowering drug as an active ingredient.

The pharmaceutical combination composition may be formulated into a general combination formulation or a timed-release combination formulation. For the timed-release combination formulation, the order of the drugs that are released may be controlled according to necessity. In one embodiment, the timed-release combination formulation may first release a statin-based lipid-lowering drug and thereafter release losartan, but the present invention is not limited thereto. The combination composition may be formulated using a method widely known by those skilled in the related art using an excipient as will be disclosed below.

Meanwhile, for the pharmaceutical composition according to the present invention, the dosage of losartan and a pharmaceutically acceptable salt thereof for treating or preventing side effects of the statin-based lipid-lowering drug may depend on the weight, age, sex and symptoms of a patient, but may generally be in the range of 0.1 to 2,000 mg, preferably 0.5 to 1,000 mg and more preferably 1.0 to 500 mg, and may be administered once or several times per day in the case of adult.

When the dosage is less than the ranges above, the effect of the losartan component on the treatment and prevention of muscle toxicity cannot be expected, and when the dosage exceeds the ranges above, the risk of generating side effects due to excessive administration is greatly increased so that the risk is beyond the effectiveness of the treatment of muscle toxicity.

Meanwhile, the pharmaceutical composition of the present invention comprising losartan and a pharmaceutically acceptable salt thereof may comprise at least one pharmaceutically acceptable carrier in addition to the active ingredients mentioned above.

The term "pharmaceutically acceptable carrier" throughout the specification refers to a pharmaceutical additive that is useful when formulated for the administration of a human body, and is non-toxic and insensitive under the conditions it is used. The specific content ratio of the carrier may be determined according to the solubility of the active ingredients, chemical properties, a route of selected administration, and also pharmaceutical practice.

More specifically, the pharmaceutical composition of the present invention may be formulated in a suitable dosage type for a desired route of administration using the pharmaceutically acceptable carriers, such as excipients, for example, a physiologically acceptable filler, disintergrant, sweetener, binder, coating agent, swelling agent, glidant, lubricant, flavor, and the like. In addition, the amount of the carrier required per dosage unit may be a sufficient amount for providing the size and administration type that can increase the adaptability toward subjects.

The method of administration may be carried out using a type of oral preparation or parenteral preparation. For example, the type may include tablets, capsules comprising particles, liquid, or powder, pills, granules, powders, troches (including liquid-filled ones), chewable tablets, multi- and nano-particles, gels, solid solutions, liposomes, films (including a mucous membrane-adhesive), ovules, sprays, and liquid medicines, but is not limited thereto. The liquid medicine may include, for example, a suspension, solution, syrup, and elixir medicine, but is not limited thereto. When formulated into a type of solid formulation, such as tablets or capsules among oral formulations, a disintergrant may be further included in addition to the active ingredient. The disintergrant may be mixed with, for example, a starch or modified starch such as sodium starch glycolate, corn starch, potato starch or pre-gelatinized starch, a clay such as bentonite, montmorillonite or Veegum, a cellulose such as microcrystal cellulose, low substituted hydroxypropyl cellulose or carboxymethyl cellulose calcium, an alginic acid such as alginate or sodium alginate, a crosslinking cellulose such as croscarmellose sodium, a gum such as guar gum or xanthan gum, a crosslinking polymer such as crospovidone, a effervescent formulation such as sodium bicarbonate or citric acid, but the present invention is not limited thereto.

The disintergrant may be comprised preferably at about 0.5wt% to about 40wt% of an administration type, and more preferably at about 1wt% to about 20wt% of an administration type, but the present invention is not limited thereto.

In addition, the binder may be further comprised in order to give adhesion. The binder that may be used herein may include, for example, microcrystal cellulose, gelatin, sugar, polyethylene glycol, natural and synthetic gums, polyvinylpyrrolidone (povidone), polyvinyl alcohol, copovidone, pre-gelatinized starch, starch, high dispersive silica, hydropropyl cellulose, hydroxypropyl methylcellulose, and the like, but the present invention is not limited thereto.

The binder may be comprised preferably at about 0.1wt% to about 40wt% of an administration type, and more preferably at about 0.5wt% to about 25wt% of an administration type, but the present invention is not limited thereto.

Also, a diluent may be further comprised. The diluent that may be used herein may include, for example, starch, microcrystalline cellulose, lactose, glucose, mannitol, alginate, alkaline earth metal salts, clays, polyethlyene glycol, calcium phosphate, and the like, but the present invention is not limited thereto.

The diluent may be comprisied preferably at about 0.5wt% to about 90wt% of an administration type, and more preferably at about 2wt% to about 75wt% of an administration type, but the present invention is not limited thereto.

Also, the lubricant may be further comprisied. The lubricant used herein may include, for example, talc, stearic acid, magnesium stearate, calcium stearate, zinc stearate, sodium stearyl fumarate, sodium lauryl sulfate, hydrogenated castor oil, polyethlyene glycol, and the like, but the present invention is not limited thereto.

The lubricant may be comprised preferably at about 0.1 wt% to about 30wt% of an administration type, and more preferably at about 0.2wt% to about 20wt% of an administration type, but the present invention is not limited thereto.

In addition, a solid-phase pharmaceutical composition may selectively comprise a surfactant, such as sodium lauryl sulfate or polysorbate 80, and a glidant, such as light anhydrous silicic acid.

Preferably, the surfactant may be comprised at about 0.1 wt% to about 30wt% of an administration type and the glidant may be comprised at about 0.2wt% to about 20wt% of an administration type, but the present invention is not limited thereto.

In addition, an antioxidant, a colorant, a flavor, a preservative, a taste masking agent, and the like may be comprised as a comprisable component.

The solid-phase pharmaceutical composition may be formed by directly pressing the contained components or pressing the components with a roller, or may be wet-, dry-, melt-granulated, melt-congealed, or extruded.

As necessary, the pharmaceutical composition may be formulated into an immediate release formulation and/or a modified release formulation according to the release aspect of the drug components, and the modified release formulation includes a delayed-, sustained-, pulsed-, controlled-, targeted- and programmed-release type.

A matrix substance that may be formulated into the modified release formulation is not limited, but at least one component selected from the group consisting of an enteric polymer, a hydrophobic material, a hydrophilic polymer, and the like, may be used as the matrix substance.

The matrix substance may be comprised preferably at about 1.0wt% to about 90.0wt% of an administration type, and more preferably at about 5.0wt% to about 70.0wt% of an administration type, but the present invention is not limited thereto.

The enteric polymer may include, for example, a polyvinyl acetate phthalate, polymethacrylate copolymer, such as, a copolymer of poly(methacrylic acid and methylmethacrylate (weight ratio of 1:1 or 1:2), and a copolymer of poly(methacrylic acid and ethylacrylate (weight ratio of 1:1), hydroxypropyl methylcellulose phthalate, hydroxypropyl methylcellulose acetate succinate, shellac, cellulose acetate phthalate, cellulose propionate phthalate, and the like, but the present invention is not limited thereto.

The hydrophobic material is a pharmaceutically acceptable salt, and for example, may include polyvinyl acetate, a polymethacrylate copolymer, such as a copolymer of poly(butyl methacrylate, 2-dimethylaminoethylmethacrylate and methyl methacrylate (weight ratio of 1:2:1), a copolymer of poly(ethylacrylate and methyl methacrylate (weight ratio of 2:1), a copolymer of poly(ethylacrylate, methyl methacrylate and trimethyl ammonioethyl methacrylate (weight ratio of 1:2:0.2 or 1:2:0.1), ethyl cellulose and cellulose acetate, fatty acids and fatty acid esters, fatty alcohols, waxes, and the like, but the present invention is not limited thereto.

More specifically, the fatty acids and fatty acid esters may include glyceryl palmitostearate, glyceryl stearate, glyceryl behenate , cetyl palmitate, glyceryl mono oleate, stearic acid, and the like and the fatty acid alcohols may include cetostearyl alcohol, cetyl alcohol, stearyl alcohol, and the like. Also, the waxes may include carnauba wax, beeswax, and the like.

The hydrophilic polymer may include, for example, sugars, cellulose derivatives, gums, proteins, polyvinyl derivatives, polyethylene derivatives, a carboxy vinyl polymer, and the like, but the present invention is not limited thereto.

More specifically, the sugars may include dextrin, polydextrin, dextran, pectin and pectin derivatives, alginate, polygalacturonic acid, xylan, arabinoxylan, arabinogalactan, starch, hydroxypropyl starch, amylose, amylopectin, and the like and the cellulose derivatives may include hydroxypropyl methylcellulose, hydroxypropyl cellulose, hydroxymethyl cellulose, hydroxyethyl cellulose, methyl cellulose, sodium carboxymethyl cellulose, and the like. The gums may include guar gum, locust bean gum, tragacantha, carrageenan, acacia gum, gum arabic, gellan gum, xanthan gum, and the like; gelatin, casein, zein, and the like may be selected and the used as the proteins; and polyvinyl derivatives may include polyvinyl alcohol, polyvinylpyrrolidone, and the like. The polyethylene derivatives may include polyethylene glycol, polyethylene oxide, and the like and the carboxyvinyl polymer may include carbomer, and the like.

In addition, the formulation of the pharmaceutical composition and the pharmaceutically acceptable carrier of the present invention may be properly selected according to techniques known in the art, but the present invention is not limited thereto.

### BRIEF DESCRIPTION OF THE DRAWINGS

The above and other objects, features and advantages of the present invention will become more apparent to those of ordinary skill in the art by describing in detail exemplary embodiments thereof with reference to the attached drawings, in which:

FIG. 1 is a graph showing CK enzyme concentration (IU/L) in the blood according to a period of administration in the experiment performed according to the example of the present invention;

FIG. 2 is a graph showing a change ratio (%) of CK enzyme concentration in the blood according to a period of administration in the experiment performed according to the example of the present invention;

FIG. 3 is a graph showing LDH enzyme concentration (IU/L) in the blood according to a period of administration in the experiment performed according to the example of the present invention; and

FIG. 4 is a graph showing a change ratio (%) of LDH enzyme concentration in the blood according to a period of administration in the experiment performed according to the example of the present invention.

### DETAILED DESCRIPTION OF EXEMPLARY EMBODIMENTS

Hereinafter, the exemplary embodiments of the present invention will be described in detail. However, the present invention is not limited to the embodiments disclosed below, but can be implemented in various forms. The following embodiments are described in order to enable those of ordinary skill in the art to embody and practice the present invention.

The terminology used herein is for the purpose of describing particular embodiments only and is not intended to be limiting the exemplary embodiments. The singular forms "a," "an" and "the" are intended to include the plural forms as well, unless the context clearly indicates otherwise. It will be further understood that the terms "comprises," "comprising," "includes" and/or "including," when used herein, specify the presence of stated features, integers, steps, operations, elements, components and/or groups thereof, but do not preclude the presence or addition of one or more other features, integers, steps, operations, elements, components and/or groups thereof.

With reference to the appended drawings, the exemplary embodiments of the present invention will be described in detail below. To aid in understanding the present invention, like numbers refer to like elements throughout the description of the figures, and the description of the same elements will be not reiterated.

**EXAMPLES**

**Experimental Example: Test of Effect of Losartan on Muscle Toxicity**

The following test was performed in order to estimate an effect of the losartan drug on preventing or inhibiting muscle toxicity.

In order to perform a comparison test of muscle toxicity according to administration of atorvastatin alone and co-administration of atorvastatin and losartan, Wistar rats (females, 300 to 350 g) were used in the experimental groups as shown in Table 1, and the experiment was performed.

**Table 1**

| Experimental Groups for Verifying Effect of Losartan on Inhibiting and Preventing Muscle Toxicity | | |
|---|---|---|
| Group | Administered Group (10 for each group) | Dosage (mg/kg) |
| 1 | Control Group | 0.5% MC, Physiological Saline |
| 2 | Atorvastatin-administered Group | 40 |
| 3 | | 80 |
| 4 | Atorvastatin + Losartan-administered Group Co-administered Group | 40 + 100 |
| 5 | | 80 + 200 |
| 6 | Atorvastatin + Losartan-administered Group Timed-release-administered Group | 80 + 200 |

For the atorvastatin-administered group, a dosage of atorvastatin was dissolved in 0.5% methyl cellulose (MC, Sigma Aldrich, USA) and then orally administered, and for the losartan-administered group, a dosage of losartan was dissolved in physiological saline and then orally administered. In addition, a 0.5% MC solution or physiological saline was orally administered to a control group.

White rats were orally administered atorvastatin, atorvastatin + losartan (co-administration), and atorvastatin + losartan (two-hours difference administration) once a day for three weeks. Then, the concentration of a muscle toxicity-associated enzyme, such as creatine kinase (CK) and lactate dehydrogenase (LDH) in blood was measured and a histological analysis was performed.

**Experimental Example 1: Measurement of Concentration of CK Enzyme in the Blood**

The CK enzyme was mainly present in the skeletal muscles or cardiac muscles with high quantity of motion. When the membrane of a muscle cell is destroyed, CK leaks all over the body thereby increasing the CK levels in the blood, and thus it can be used as a marker to diagnose musculoskeletal toxicity. Accordingly, CK concentration in the blood was measured in order to confirm whether losartan inhibits muscle toxicity side effects caused by atorvastatin.

CK concentration in the blood was measured by analyzing serums three times at 0 weeks, 1 week, and 2 weeks after starting the administration. An increase rate of concentration in the blood was calculated using the following equation and the results are shown.

Change rate of concentration in blood (%) = (Concentration in blood of the testing group - concentration in blood of the control group)/concentration in blood X 100

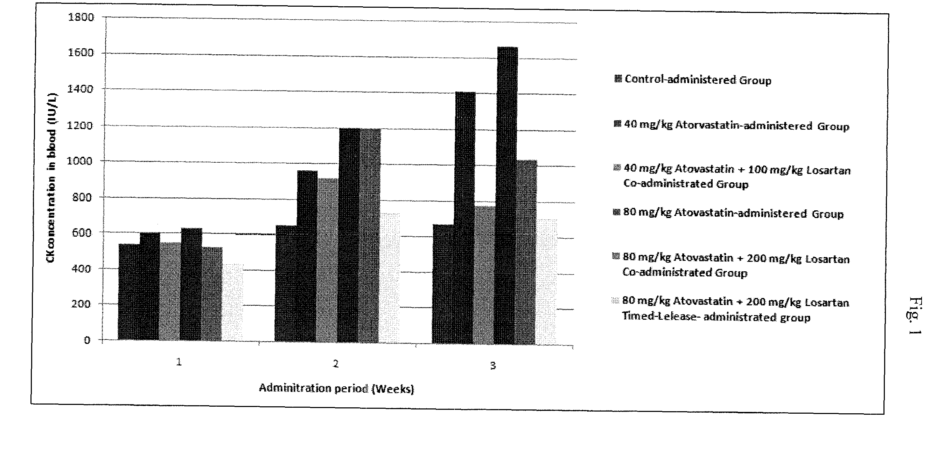

As a result, the measured CK concentrations in the blood are listed in Table 2 and shown in FIG. 1.

**Table 2**

| Change of Concentration of CK Enzyme in the Blood according to the Administration of the Losartan Drug | | | |
|---|---|---|---|
| Administered Groups | CK Enzyme Concentration in the Blood According to the Administration Period (IU/L) | | |
| | 1 week | 2 weeks | 3 weeks |
| Control-administered Group | 538.6±49.7 | 650.1±44.5 | 667.4±50.0 |
| 40 mg/kg Atorvastatin-administered Group | 600.8±67.2 | 958.8±54.4 | 1407.9±117.5 |
| 40 mg/kg Atorvastatin + 100mg/kg Losartan Co-administered Group | 549.3±45.7 | 920.9±131.7 | 775.8±93.9 |
| 80 mg/kg Atorvastatin-administered Group | 632.2±83.2 | 1202.8±138.4 | 1664.6±149.9 |
| 80 mg/kg Atorvastatin + 200 mg/kg Losartan Co-administered Group | 531.1±70.8 | 1198.3±110.9 | 1038.0±264.2 |
| 80 mg/kg Atorvastatin + 200 mg/kg Losartan Timed-release-administered Group | 434.1±35.9 | 728.9±63.7 | 703.3±109.5 |

It could be confirmed that the longer the administration period and the larger the dosage, the more the CK concentration in the blood increased. Meanwhile, it could be confirmed that the CK concentration ceased to increase, and also the CK concentration actually decreased when atorvastatin was administered along with the losartan drug, especially, after three weeks of administration.

As shown above in Table 2, it could be confirmed that when the atorvastatin drug was continuously administered, CK enzymes continuously increased, whereas CK enzymes decreased when administered along with the losartan drug.

When CK enzyme concentration in the blood was compared after three weeks of administration, it had decreased by 44.8% for the co-administered group of 40 mg atorvastatin and 100 mg losartan, 37.6% for the co-administered group of 80 mg atorvastatin and 200 mg losartan, and 57.7% for a Timed-release-administered group of 80 mg atorvastatin and 200 mg losartan as compared with the single administration of atorvastatin.

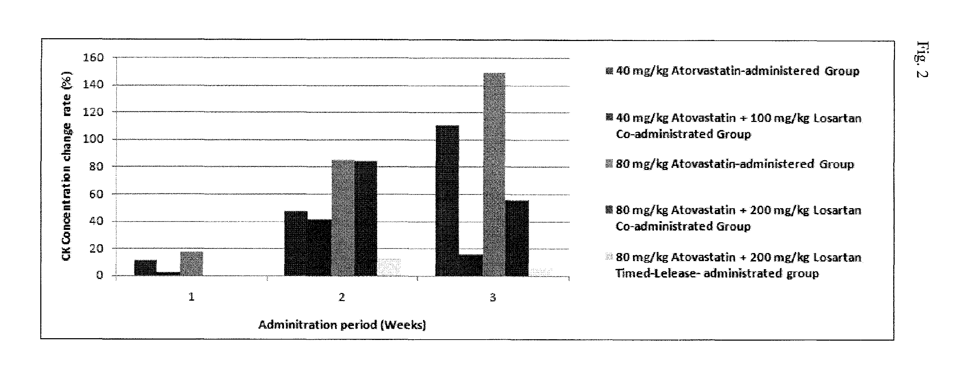

The following Table 3 and FIG. 2 show a change rate of CK enzyme concentrations in the blood according to the administration of the losartan drug by calibration based on the control group, which confirms a clearer therapeutic effect.

**Table 3**

| Change Rate of CK Enzyme Concentration in the Blood According to the Administration of Losartan Drug (Calibration based on the Control Group) | | | |
|---|---|---|---|
| Administered Groups | Change Rate of CK Enzyme Concentration in the Blood According to the Administration Period As Compared With the Control Group (%) | | |
| | 1 week | 2 weeks | 3 weeks |
| 40 mg/kg Atorvastatin administered Group | 11.5 | 47.5 | 110.5 |
| 40 mg/kg Atorvastatin + 100 mg/kg Losartan Co-administered Group | 2.0 | 41.7 | **16.2** |
| 80 mg/kg Atorvastatin-administered Group | 17.4 | 85.0 | 149.4 |
| 80 mg/kg Atorvastatin + 200 mg/kg Losartan Co-administered Group | -1.4 | 84.3 | **55.5** |
| 80 mg/kg Atorvastatin + 200 mg/kg Losartan Timed-release-administered Group | -19.4 | 12.1 | **5.4** |

As shown in Table 3 and FIG. 2, it could be confirmed that a change rate (%) of CK concentration in the blood that had been increased by administering atorvastatin was significantly decreased when co-administered along with losartan drug after three weeks of starting the administration.

As shown in Table 3, a change rate (%) of CK concentration in the blood was decreased by 85.3% for the co-administered group of 40 mg atorvastatin and 100 mg losartan as compared with the single-administered group of 40 mg atorvastatin, and a change rate (%) of CK concentration in the blood was decreased by 62.9% for the co-administered group of 80 mg atorvastatin and 200 mg losartan as compared with the single-administered group of 80 mg atorvastatin. And also, a better effect was exhibited by a decrease of up to 96.4% for the timed-release-administered groups of 80 mg atorvastatin and 200 mg losartan, respectively as compared with the single-administered group of 80 mg atorvastatin. Accordingly, it was confirmed that the losartan drug can treat and prevent side effects such as muscle toxicity generated by a statin-based lipid-lowering drug.

**Experimental Example 2: Measurement of Lactate Dehydrogenase (LDH) Enzyme Concentration in the Blood**

LDH enzyme, which is another factor that confirms muscle toxicity, is plentifully found in many body tissues, such as the heart, liver, kidneys, skeletal muscles, brain, blood cells, and lungs, and it is widely used as a marker of tissue damage because LDH enzyme in a tissue cell flows out from a cell due to the change or destruction of cytopermeability and the like, thereby increasing the LDH level in the blood. Accordingly, LDH concentration in the blood was measured by using the same method as CK in order to confirm whether losartan inhibits side effects such as muscle toxicity caused by atorvastatin.

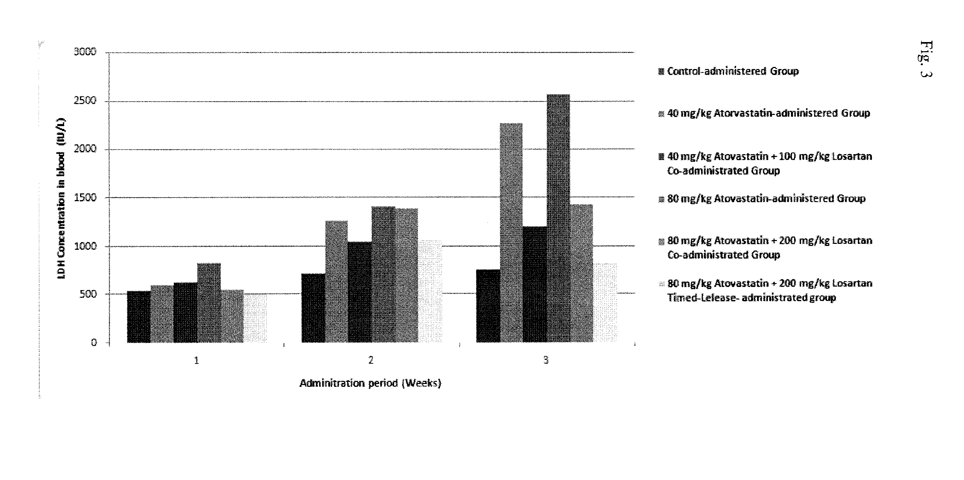

As analyzed results, LDH enzyme concentrations in the blood of each experimental group are shown in Table 4 and FIG. 3.

**Table 4**

| Change of LDH Enzyme Concentration in the Blood According to the Administration of the Losartan Drug | | | |
|---|---|---|---|
| Administered Groups | LDH Enzyme Concentration in the Blood According to the Administration Period (IU/L) | | |
| | 1 week | 2 weeks | 3 weeks |
| Control-administered Group | 547.3±99.6 | 724.0±47.2 | 756,3±88.5 |
| 40 mg/kg Atorvastatin-administered Group | 598.4±48.9 | 1259.6±125.9 | 2268.2±226.8 |
| 40 mg/kg Atorvastatin + 100mg/kg Losartan Co-administered Group | 635.7±56.9 | 1050.2±214.1 | 1202.1±165.0 |
| 80 mg/kg Atorvastatin-administered Group | 824.9±109.4 | 1411.6±161.3 | 2561.4±251.2 |
| 80 mg/kg Atorvastatin + 200 mg/kg Losartan Co-administered Group | 556.8±50.3 | 1394.4±165.3 | 1434.2±274.4 |
| 80 mg/kg Atorvastatin + 200 mg/kg Losartan Timed-release-administered Group | 508.0±58.6 | 1059.4±127.4 | 823.5±105.4 |

LDH enzyme concentration was also the same as that of CK enzyme. It was confirmed that the longer the atorvastatin-administration period and the larger the dosage of atorvastatin, the more the LDH concentration in the blood increased. Meanwhile, it was confirmed that LDH enzyme concentration slowly increased when atorvastatin was co-administered along with losartan.

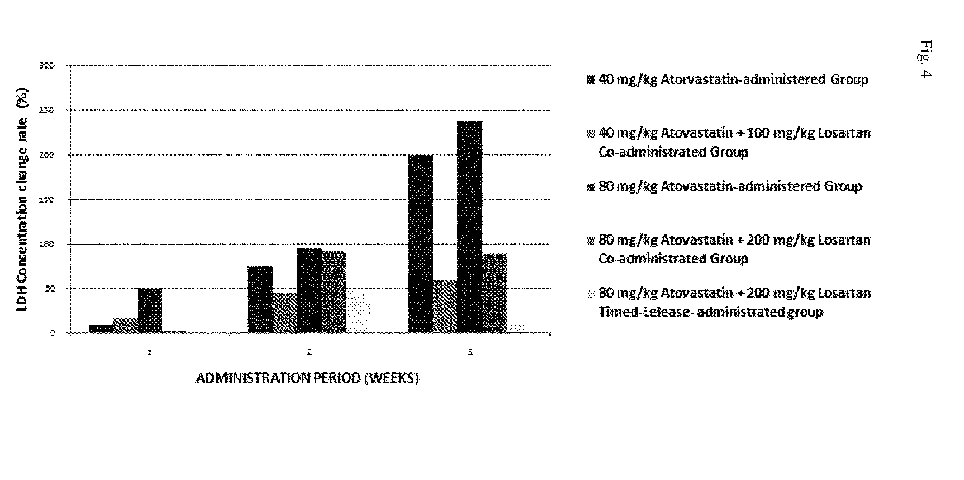

The following Table 5 and FIG. 4 show a change rate of LDH enzyme concentrations in the blood according to the losartan drug administration by calibration based on the control group, which confirms a clearer therapeutic effect.

**Table 5**

| Change Rate of LDH Enzyme Concentration in the Blood According to the Administration of the Losartan Drug (As Compared with the Control Group) | | | |
|---|---|---|---|
| Administered Groups | Change Rate of LDH Enzyme Concentration in the Blood According to the Administration Period As Compared With the Control Group (%) | | |
| | 1 week | 2 weeks | 3 weeks |
| 40 mg/kg Atorvastatin-administered Group | 9.3 | 74.0 | 199.9 |
| 40 mg/kg Atorvastatin + 100 mg/kg Losartan Co-administered Group | 16.2 | 45.1 | **58.9** |
| 80 mg/kg Atorvastatin-administered Group | 50.7 | 95.0 | 238.7 |
| 80 mg/kg Atorvastatin + 200 mg/kg Losartan Co-administered Group | 1.7 | 92.6 | **89.6** |
| 80 mg/kg Atorvastatin + 200 mg/kg Losartan Timed-release-administered Group | -7.2 | 46.3 | **8.9** |

As shown in Table 5 and FIG. 4, it could be confirmed that a change rate (%) of LDH concentration in the blood that was increased when atorvastatin was administered was significantly decreased when co-administered along with the losartan drug after three weeks of starting the administration. As shown in Table 5, it was surprisingly found that a change rate (%) of LDH concentration in the blood was decreased by 70.5% for the co-administered group of 40 mg atorvastatin and 100 mg losartan as compared with the single administration of 40 mg atorvastatin and a change rate (%) of LDH concentration in the blood was decreased by 62.5% for the co-administered group of 80 mg atorvastatin and 200 mg losartan as compared with the single administration of 80 mg atorvastatin. In addition, a better effect was exhibited by a decrease of up to 96.3% for the Timed-release-administered group of 80 mg atorvastatin and 200 mg losartan, respectively, as compared with the single-administered group of 80 mg atorvastatin. The result was also surprising in that it verified that thelosartan drug can treat and prevent the side effects by decreasing side effects such as muscle toxicity caused by a statin-based lipid-lowering drug.

**Example 3: Histological Analysis of the Degree of Muscle Damage**

A histological analysis was performed as another experiment in order to confirm whether losartan inhibits side effects such as muscle toxicity caused by atorvastatin.

The rats were anesthetized with ether after three weeks of starting the administration for each experimental group; an extensor digitorum longus muscle was removed; fixed in paraffin; and then made into 5 µm coronal sections. The paraffin was removed with xylene, hydrophilicized with alcohol, and then stained with hematoxylin and eosin to observe the tissue through an optical microscope (Olympus Optical, Co., Japan). In addition, the degree of histological damage of the extensor digitorum longus muscle that was cut lengthwise in a double-blind test was scored as shown in the following Table 6 using the method as described in documents, such as F. Russell, J. Seachrist, and the like.

**Table 6**

| Histological lesion Type | Score |
|---|---|
| Normal | 0 |
| Internal nuclei | +1 |
| Myositis | +2 |
| Internal nuclei + Myositis | +3 |

The experiment results are shown in the following Table 7.

**Table 7**

| Degree of Muscle Tissue Damage According to the Administration of the Losartan Drug (After three weeks) | |
|---|---|
| Administered Groups | Scores (0: Normal, +1: internal nuclei, +2: Myositis, +3: internal nuclei + myositis) |
| 40 mg/kg Atorvastatin-administered Group | 1.0±0.0 |
| 40 mg/kg Atorvastatin + 100 mg/kg Losartan Co-administered Group | 0.6±0.3 |
| 80 mg/kg Atorvastatin-administered Group | 1.8±0.2 |
| 80 mg/kg Atorvastatin + 200 mg/kg Losartan Co-administered Group | 0.5±0.3 |
| 80 mg/kg Atorvastatin + 200 mg/kg Losartan Timed-release-administered Time-lapse-administered Group | 0.3±0.2 |

The degree of tissue damage was directly investigated after three weeks of drug administration. As a result, it was confirmed that atorvastatin and losartan showed an effect of preventing tissue damage when administered at the same time, like CK and LDH enzyme concentrations in the blood as mentioned above.

As shown in Table 7, there was a surprising effect that the degree of damage to the muscle tissue was decreased by about 40.0% for the co-administered group of 40 mg atorvastatin and 100 mg losartan as compared with the single administration of 40 mg atorvastatin and the degree of damage to the muscle tissue was decreased by about 72.2% for the co-administered group of 80 mg atorvastatin and 200 mg losartan as compared with the single administration of 80 mg atorvastatin, In addition, a better effect was exhibited by a decrease of up to about 83.3% for the timed-release-administered group of 80 mg atorvastatin and 200 mg losartan as compared with the single administration group of 80 mg atorvastatin. As shown in the results of directly measuring the degree of muscle damage, it was confirmed that the losartan drug can treat and prevent the side effects such as muscle toxicity caused by a statin-based lipid-lowering drug.

From the above-mentioned results, it could be surprisingly confirmed that losartan drug has an effect of treating or preventing muscular side effects such as myositis, myopathy, rhabdomyolysis and myalgia caused by taking a statin-based lipid-lowering drug such as atorvastatin.

**Preparation Example 1: Preparation of Tablet Comprising 10 mg of Losartan Potassium**

100.0 g of losartan potassium 234.0 g of microcrystalline cellulose, 146.0 g of lactose hydrate, and 50.0 g of pre-gelatinized starch were each sieved through a No. 20 sieve, and then mixed in a V-type mixer (Cheil Company, Korea) for 20 minutes. Finally, 10.0 g of stearic acid was sieved through a No. 35 sieve, added to the mixture, and then mixed for 3 minutes. Subsequently, the final mixture was prepared into a tablet comprising 10.0 mg of losartan potassium per tablet (each having a weight of 54.0 mg), and then the tablet was injected into a coating machine (SFC-30N, Sejong Machinery, Korea). Separately, a coating solution was prepared by dissolving 4.0 g of polyethylene glycol and 36.0 g of a coating agent hydroxypropyl methylcellulose in an 80% ethanol solution, and the tablet was coated with the coating solution to prepare the tablet comprising 10.0 mg of losartan potassium.

**Preparation Example 2: Preparation of Tablet Comprising 20 mg of Losartan Potassium**

The tablet was prepared with the same method as in Preparation Example 1, except that the method was performed to prepare a tablet weighing 108.0 mg.

**Preparation Example 3: Preparation of Tablet Comprising 50 mg of Losartan Potassium**

The tablet was prepared with the same method as in Preparation Example 1, except that the method was performed to prepare a tablet weighing 270.0 mg.

**Preparation Example 4: Preparation of Tablet Comprising 100 mg of Losartan Potassium**

The tablet was prepared with the same method as in Preparation Example 1, except that the method was performed to prepare a tablet weighing 540.0 mg.

**Preparation Example 5: Preparation of Tablet Comprising 200 mg of Losartan Potassium**

200.0 g of losartan potassium, 234.0 g of microcrystalline cellulose, 146.0 g of lactose hydrate, and 50.0 g of pre-gelatinized starch were each sieved through a No. 20 sieve, and then mixed in a V-type mixer (Cheil Company, Korea) for 20 minutes. Finally, 10.0 g of stearic acid was sieved through a No. 35 sieve, added to the mixture, and then mixed for 3 minutes. Subsequently, the final mixture was made to prepare a tablet comprising 200.0 mg of losartan potassium per tablet (each having a weight of 640.0 mg), and then the tablet was injected into a coating machine (SFC-30N, Sejong Machinery, Korea). Separately, a coating solution was prepared by dissolving 5.0 g of polyethylene glycol and 35.0 g of a coating agent hydroxypropyl methylcellulose in an 80%-ethanol solution, and the tablet was coated with the coating solution to prepare the tablet comprising 200.0 mg of losartan potassium.

**Preparation Example 6: Preparation of Losartan Potassium-Atorvastatin Combination Tablet**

1) Preparation of Losartan Potassium Granule: 50.0 g of losartan potassium, 215.0 g of microcrystalline cellulose, 240.0 g of lactose hydrate, and 20.0 g of colloidal silicon dioxide were sieved through a No. 20 sieve, respectively, and then mixed in a high speed mixer (YC-SMG-10J, Yenchen, Taiwan) for 2 minutes. Separately, 13.5 g of povidone K-30 was dissolved in ethanol to prepare a binding solution, and then the binding solution was added to the high speed mixer to mix for 3 minutes. After completing the mixture, it was dried in a hot-water drier at 60 °C for 4 hours and then again sieved through a No. 20 sieve. 8.0 g of colloidal silicon dioxide and 40.0 g of crospovidone were added to the granule, and then mixed in a V type-mixer (Cheil Company, Korea) for 10 minutes. Finally, 13.5 g of magnesium stearate was sieved through a No. 35 sieve and mixed into the mixture for 3 minutes to prepare a losartan potassium-containing granule.

2) Preparation of Atorvastatin Granule: 207.2 g of atorvastatin calcium salt, 450.0 g of precipitated calcium carbonate, 950.0 g of microcrystalline cellulose, 222.8 g of D-mannitol, 385.0 g of low substituted hydroxylpropyl cellulose, and 250.0 g of sodium starch glycolate were each sieved through a No. 20 sieve, and then mixed in high speed mixer (YC-SMG-10J, Yenchen, Taiwan) for 2 minutes. Separately, 35.0 g of hydroxypropyl cellulose and 10.0 g of polysorbate 80 were dissolved in ethanol to prepare a binding solution, and then the binding solution was added to the high-speed mixer to mix for 3 minutes. After completing the mixture, it was dried in a hot-water drier at 60 °C for 4 hours and then again sieved through a No. 20 sieve. 225.0 g of microcrystalline cellulose, 25.0 g of colloidal silicon dioxide and 115.0 g of sodium starch glycolate were added to the granule, and then mixed in a V type-mixer (Cheil Company, Korea) for 10 minutes. Finally, 25.0 g of magnesium stearate was sieved through a No. 35 sieve and mixed into the mixture for 3 minutes to prepare an atorvastatin-containing granule.

3) Preparation of Combination Tablet: The losartan potassium granule of the above 1) and the atorvastatin granule of the above 2) were mixed in a V type-mixer (Cheil Company, Korea), the tablet was prepared to comprise 10.0 mg of losartan potassium and 41.44 mg of atorvastatin calcium per tablet (weight of one table: 700.0 mg), and then the tablet was injected into a coating machine (SFC-30N, Sejong Machinery, Korea). Separately, a coating solution was prepared by dissolving 10.0 g of polyethylene glycol and 65.0 g of a coating agent hydroxypropyl methylcellulose in 750.0 g of 80%-ethanol solution, and the tablet was coated with the coating solution to prepare the combination tablet comprising 10.0 mg of losartan potassium and 41.44 mg of atorvastatin calcium salt.

**Preparation Example 7: Preparation of Losartan Potassium-Atorvastatin-containing Timed-release Combination Tablet**

1) Preparation of losartan potassium Inner-core Tablets: A losartan potassium granule was prepared with the same method as that of Preparation Example 6, but the prepared granule was prepared into a tablet with a 6.0 mm circle punch to comprise 10.0 mg of losartan potassium per tablet (120-mg tablet). Separately, the coating solution was prepared by dissolving 65 g of hydroxypropyl methylcellulose and 10.0 g of triethyl citrate in 750 g of ethanol and 750 g of methylene chloride. The tablet comprising 10.0 mg of losartan potassium prepared as mentioned above was injected into a coating machine (SFC-30N, Sejong Machinery, Korea) and then coated with the coating solution to prepare losartan potassium-containing inner core tablets.

2) Preparation of Atorvastatin Granule: An atorvastatin granule was prepared with the same method as that of Preparation Example 6.

3) Preparation of Cored Tablets: The losartan potassium layered tablets of the above 1) (135.0-mg tablets) and the atorvastatin granule of the above 2) (585.0 mg per tablet) were injected into a core tableting machine (RUD-1, Kilian, Germany) and then the tablet was prepared to contain 10.0 mg of losartan potassium and 41.44 mg of atorvastatin calcium per tablet (weight of one tablet: 720.0 mg) so that atorvastatin was released first because it was on the outside layer and losartan potassium was released later because it was in the nucleus. The tablet was injected into a coating machine (SFC-30N, Sejong Machinery, Korea). Then, separately, a coating solution was prepared by dissolving 10.0 g of polyethylene glycol and 65 g of a coating agent hydroxypropyl methylcellulose in 750.0 g of 80% ethanol solution, and the tablet was coated with the coating solution to prepare the timed-release combination tablet comprising 10.0 mg of losartan potassium and 41.44 mg of atorvastatin calcium salt.

**Preparation Example 8: Preparation of Losartan Potassium-Atorvastatin-Containing Timed-release Combination Tablet**

Preparation of Losartan Potassium Inner-core Tablets: 500.0 g of losartan potassium, 45.0 g of microcrystalline cellulose, 20.0 g of lactose hydrate, and 5.0 g of colloidal silicon dioxide were each sieved through a No. 20 sieve, and then mixed in high speed mixer (YC-SMG-10J, Yenchen, Taiwan) for 2 minutes. Separately, 5.0 g of povidone was dissolved in ethanol to prepare a binding solution, and then the binding solution was added to the high speed mixer to mix for 3 minutes. After completing the mixture, it was dried in a hot-water drier at 60 °C for 4 hours and then again sieved through a No. 20 sieve. 5.0 g of colloidal silicon dioxide and 10.0 g of crospovidone were added to the granule, and then mixed in a V type-mixer (Cheil Company, Korea) for 10 minutes. Finally, 10.0 g of magnesium stearate was sieved through a No. 35 sieve and mixed into the mixture for 3 minutes to prepare a losartan potassium-containing granule. The prepared granule was prepared into a tablet with a 6.0-mm circle punch to contain 100.0 mg of losartan potassium per tablet (120 mg per one tablet). Separately, a coating solution was prepared by dissolving 10.0 g of polyethylene citrate and 65 g of a coating agent hydroxypropyl methylcellulose in 750.0 g of 80% ethanol solution. The tablet containing 10.0 mg of losartan potassium as mentioned above was injected into a coating machine (SFC-30N, Sejong Machinery, Korea) and coated with the coating solution to prepare a losartan potassium-containing inner-core tablet.

The following processes were performed with the same method as Preparation Example 7 to prepare a timed-release combination tablet, which contains 100.0 mg of losartan potassium and 41.44 g of g of atorvastatin calcium salt.

According to the present invention, a pharmaceutical composition comprising losartan or a pharmaceutically acceptable salt thereof provides an effect of treating or preventing muscle-related side effects that may be generated by taking a statin-based drug for treating hyperlipidemia, as well as an intrinsic pharmacological effect of treating or preventing hypertension. The pharmaceutical composition shows effects of effectively and significantly decreasing and preventing side effects such as muscle toxicity when co-administered with a statin-based drug as well as when administered in sequence with a statin-based drug.

While the invention has been shown and described with reference to certain exemplary embodiments thereof, it will be understood by those skilled in the art that various changes in form and details may be made therein without departing from the scope of the invention as defined by the appended claims.

## Claims

1. A pharmaceutical composition for treating or preventing muscular side effects caused by a statin-based lipid-lowering drug, comprising losartan or a pharmaceutically acceptable salt thereof as an active ingredient.

2. The pharmaceutical composition of claim 1, wherein the losartan or pharmaceutically acceptable salt thereof is administered with the statin-based lipid-lowering drug at the same time or in sequence.

3. The pharmaceutical composition of claim 1, wherein the pharmaceutical composition further provides a medicinal use for treating or preventing hypertension.

4. The pharmaceutical composition of claim 1, wherein the muscular side effects caused by the statin-based lipid-lowering drug include at least one selected from the group consisting of myositis, myopathy, rhabdomyolysis and myalgia.

5. The pharmaceutical composition of claim 1, wherein the statin-based lipid-lowering drug is at least one selected from the group consisting of simvastatin, lovastatin, atorvastatin, pitavastatin, rosuvastatin, fluvastatin, pravastatin, and pharmaceutically acceptable salts thereof.

6. The pharmaceutical composition of claim 1, wherein the total dosage of the losartan or pharmaceutically acceptable salt thereof is 0.1 mg to 2,000 mg a day.

7. The pharmaceutical composition of claim 1, wherein the total dosage of the losartan or pharmaceutically acceptable salt thereof is 0.5 mg to 1,000 mg a day.

8. The pharmaceutical composition of claim 1, wherein the total dosage of the losartan or pharmaceutically acceptable salt thereof is 1.0 mg to 500 mg a day.

9. The pharmaceutical composition of claim 1, wherein the losartan or a pharmaceutically acceptable salt thereof is losartan potassium salt.

10. A pharmaceutical composition for treating or preventing muscular side effects caused by a statin-based lipid-lowering drug, and decreasing lipids, comprising losartan or a pharmaceutically acceptable salt thereof, and the statin-based lipid-lowering drug as active ingredients.

11. The pharmaceutical composition of claim 10, wherein the pharmaceutical composition further provides a medicinal use for treating or preventing hypertension.

12. The pharmaceutical composition of claim 10, wherein the pharmaceutical composition is formulated into a general combination formulation or a timed-release combination formulation.

13. The pharmaceutical composition of claim 10, wherein the timed-release combination formulation first releases the statin-based lipid-lowering drug and then later releases the losartan.
